# EUROPÄISCHE PATENTSCHRIFT

(11) **EP 2 452 653 B1**
(45) Veröffentlichungstag und Bekanntmachung des Hinweises auf die Patenterteilung: **27.11.2013**
(21) Anmeldenummer: 11185356.0
(22) Anmeldetag: 17.10.2011
(51) Int. Cl.: A61F 2/00, A61K 38/12, C07K 7/64

(54) **cRGD-Peptid-Derivat und seine Herstellung sowie Implantat mit einer Beschichtung, die ein cRGD-Peptid-Derivat enthält**
cRGD peptide derivative and its manufacture and implant having a coating containing a cRGD peptide derivative
Dérivé de peptide cRGD et sa fabrication ainsi qu'implant doté d'un revêtement comprenant un dérivé de peptide cRGD

(30) Priorität: 12.11.2010 US 412798 P
(43) Veröffentlichungstag der Anmeldung: 16.05.2012
(73) Patentinhaber: Biotronik AG, 8180 Bülach (CH)
(72) Erfinder: Borck, Alexander, 91086 Aurachtal (DE)
(74) Vertreter: Lindner-Vogt, Karin L.

(56) Entgegenhaltungen:
- WO-A2-2007/146001
- WO-A2-2010/002584
- ZHAN C ET AL: "Cyclic RGD conjugated poly(ethylene glycol)-co-poly(lactic acid) micelle enhances paclitaxel anti-glioblastoma effect", JOURNAL OF CONTROLLED RELEASE, ELSEVIER, AMSTERDAM, NL, Bd. 143, Nr. 1, 2. April 2010 (2010-04-02) , Seiten 136-142, XP026964332, ISSN: 0168-3659 [gefunden am 2010-01-07]
- KIM ET AL: "Self-assembled glycol chitosan nanoparticles for the sustained and prolonged delivery of antiangiogenic small peptide drugs in cancer therapy", BIOMATERIALS, ELSEVIER SCIENCE PUBLISHERS BV., BARKING, GB, Bd. 29, Nr. 12, 4. März 2008 (2008-03-04), Seiten 1920-1930, XP022499090, ISSN: 0142-9612, DOI: 10.1016/J.BIOMATERIALS.2007.12.038
- NG JECK FEI ET AL: "Cationized bovine serum albumin with pendant RGD groups forms efficient biocoatings for cell adhesion.", JOURNAL OF BIOMEDICAL MATERIALS RESEARCH. PART B, APPLIED BIOMATERIALS NOV 2011 LNKD- DOI:10.1002/JBM.B.31897 PUBMED:21793202, Bd. 99, Nr. 2, November 2011 (2011-11), Seiten 282-290, XP002670128, ISSN: 1552-4981

## Beschreibung

Die Erfindung betrifft ein cRGD-Peptid-Derivat nach Anspruch 1 und ein dazugehöriges Herstellungsverfahren sowie ein Implantat mit einer Beschichtung, die ein cRGD-Peptid-Derivat enthält.

### Stand der Technik und Hintergrund der Erfindung

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper, durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder TiA16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem beispielsweise der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Beschichtung bereitgestellt werden.

Es ist ferner bekannt, dass die Triade RGD (Arg-Gly-Asp) vielen Integrinen als eine primäre Erkennungssequenz für Proteine der extrazellulären Matrix dient. Peptide, welche diese Sequenz enthalten, können somit die Liganden dieser Integrine nachahmen und daher an diese binden. Lineare RGD-Peptide zeigen eine niedrige Affinität zu vielen Integrinen, jedoch führt eine Kopf-Schwanz-Cyclisierung bei Pentapeptiden zu einer konformationellen Einschränkung, so dass die Bindungsfähigkeit zu einigen Integrinen erhöht wird. Bei entsprechender Auswahl der Aminosäuren, die die RGD-Sequenz flankieren, können Peptide synthetisiert werden, die selektiv an bestimmte Integrine bzw. Integringruppen binden und diese somit inhibieren. Aufgrund der Tatsache, dass RGD-Peptide selektive Antagonisten für Integrine sind, werden sie bzw. von ihnen abgeleitete Peptidomimetika hinsichtlich ihrer medizinischen Relevanz erforscht. So wird z.B. das Integrin α_{υ}β₃, das sehr häufig von Tumorzellen exprimiert wird und eine Schlüsselrolle in dem Mechanismus des invasiven Tumorwachstums besitzt, von dem Peptid c(RGDfV) gut inhibiert. Weiterhin werden cRGD-Peptide als Inhibitoren für die Angiogenese bei Tumorerkrankungen und bei der Osteoporose eingesetzt. Eine Übersicht zum Wirkmechanismus von cRGDs und der Funktion von Integrinen kann der Dissertation von Kai Jenssen, "Peptide und peptidomimetische Verbindungen als Werkzeuge in der Proteomanalyse", Bielefeld 2004, entnommen werden.

Es ist ferner bekannt, nanoskalige Polymermizellen endständig mit cRDG-Peptiden zur Verbesserung der Aufnahme in Tumorzellen zu funktionalisieren (Norased Nasongkla et al. "cRGD-Funktionalized Polymer Micelles for Targeted Doxorubicin Delivery", Angew. Chem. Int. Ed. 2004, 43, 6323-6327).

Weiterhin ist bekannt, Polymer-Stents mit Integrin-bindenden cRGD-Peptiden zur Reduktion der neointimalen Hyperplasie durch Attraktion von endothelialen Progenitorzellen zu beladen (Pressemitteilung der Deutschen Gesellschaft für Kardiologie - Herzund Kreislaufforschung e. V. (DGK), September 2005).

WO2010002584 offenbart cRGD-Polyesteramid-Verbindungen welche zusammen mit Everolimus (Wirkstoff) als eine Monoschicht auf ein Implantat (Stent) appliziert werden.

WO2007146001 offenbart funktionalisierte cRGDf/yK -Verbindungen die sich durch eine Aspartat-Seitenkette, einen anderen Linker als poly-Lysin sowie durch das Fehlen eines hydrophoben Rests von den beanspruchten Verbindungen der Formel (1) unterscheiden.

Für die Entwicklung wirkstoffbeschichteter Implantate, insbesondere wirkstoffbeschichteter Stents (sogenannter DES-Stents), stellt der Einsatz von cRGDs somit einen vielversprechenden Ansatz zur Verbesserung der Verträglichkeit der Implantate dar. Der Einsatz von cRGDs ist jedoch vornehmlich zur Verbesserung des Einheilungsprozesses notwendig. Für eine langfristig antiproliferative Wirkung ist es in der Regel dennoch notwendig, andere Wirkstoffe, z.B. Rapamycin, aus der Beschichtung freizusetzen. Damit besteht bei nachträglicher Aufnahme von cRGD in ein bestehendes Beschichtungssystem das Problem, dass zum einen sichergestellt werden muss, dass cRGDs zu einem möglichst frühen Zeitpunkt an der Oberfläche des Implantats bereitgestellt werden, sich jedoch andererseits die Elutionscharakteristik eines sich in einer darunter befindlichen Basis der Beschichtung bereitgestellten Wirkstoffs nicht verändern soll. Ansonsten wäre eine erneute Optimierung der Elutionscharakteristik des Wirkstoffs erforderlich, was im Einzelfall sehr aufwendig ist und eine Variation des Systems erschwert.

Es wäre daher von Vorteil, die Bereitstellung von cRGDs an der Implantatoberfläche so zu gestalten, dass sich die Elutionscharakteristik aus der darunter liegenden Basisschicht für einen dort eingebetteten Wirkstoff nicht oder nur unwesentlich ändert. Ferner wäre von Vorteil, wenn die Erkennungssequenzen der cRDDs sofort nach Implantation zugänglich sind, also zum Beispiel nicht erst durch allmähliche Degradation der Beschichtungsmatrix freigelegt werden.

### Zusammenfassung der Erfindung

Ein Aspekt der Erfindung liegt in der Bereitstellung eines cRGD-Peptid-Derivats der Formel (1): 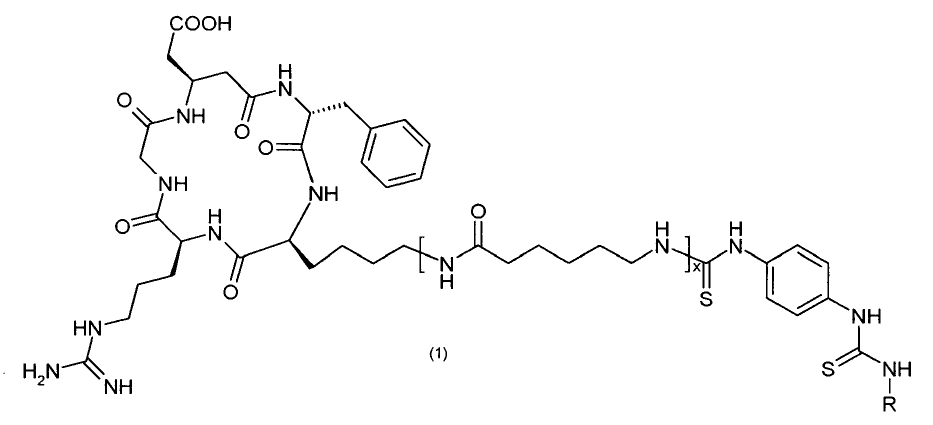 mit x = 0 - 8, insbesondere 4 - 8, und R einem hydrophoben Rest.

Vorzugsweise ist der hydrophobe Rest R derart beschaffen ist, dass ein den gleichen Rest aufweisendes primäres Amin R-NH₂ einen logP-Wert von größer als -0.2, insbesondere größer als 1.9 aufweist. Der P-Wert oder auch Octanol/Wasser-Verteilungskoeffizient ist ein dimensionsloser Verteilungskoeffizient, der das Verhältnis der Konzentrationen einer Chemikalie in einem Zweiphasensystem aus 1-Octanol und Wasser angibt (bei 25°C). P wird in der Regel in der Form des dekadischen Logarithmus als Log P angegeben.

Der Erfindung liegt die Erkenntnis zugrunde, dass ein im vorgenannten Sinne funktionalisiertes cRGD-Peptid aufgrund des hydrophoben Restes durch Selbstorganisation eine Monoschicht auf der hydrophoben Basis einer Implantatsbeschichtung bilden kann. Die hydrophobe Domäne des Peptids ragt dabei in die hydrophobe Basis der Beschichtung, sodass die Erkennungssequenzen der cRGDs nach außen gerichtet sind.

Ferner steht R vorzugsweise für einen Rest ausgewählt aus der Gruppe umfassend substituierte oder unsubstituierte aliphatische Kohlenwasserstoffe (zum Beispiel ein Alkylrest, wie Heptyl, oder ein halogenierter Alkylrest); substituierte oder unsubstituierte heteroaliphatische Kohlenwasserstoffe mit wenigstens einen Heteroatom ausgewählt aus N, O, S oder P; substituierte oder unsubstituierte Aromaten (zum Beispiel ein 2-Anthracyl- oder 4-Heptylarylrest); und substituierte oder unsubstituierte N-, O-, S- oder P-Heteroaromaten.

Alternativ steht R für eine polymere Gruppe, ausgewählt aus Chitosan, Polylysin (vorzugsweise Poly-D-Lysin), Polylactid-co-glycolid (PLGA) und Polylactid (PLA).

Ein weiterer Aspekt der Erfindung liegt in der Bereitstellung eines Implantats, vorzugsweise Stents, mit einer Beschichtung, die das obengenannte cRGD-Peptid-Derivat enthält. Insbesondere umfasst diese Beschichtung:
(i) eine Basis aus einem hydrophoben Material, vorzugsweise einen Wirkstoff enthaltend; und
(ii) eine Deckschicht, die das cRGD-Peptid-Derivat enthält oder aus diesem besteht.

Nach einer bevorzugten Ausführungsform ist vorgesehen, dass das hydrophobe Material der Basis Polylactid-co-glycolid (PLGA) oder Polylactid (PLA) enthält und das cRGD-Peptid-Derivat in der Deckschicht einen hydrophoben polymeren Rest aus Polylactid-co-glycolid (PLGA) beziehungsweise Polylactid (PLA) aufweist. Das Molekulargewicht der polymeren Gruppe kann insbesondere mit Hinsicht auf das Material der Basis der Implantatsbeschichtung vorgegeben werden, d. h. Material der Basis und des funktionalisierten cRGDs werden aufeinander abgestimmt und enthalten zum Beispiel beide Polylactid-Fragmente gleicher Kettenlänge.

Diesem Aspekt der Erfindung liegt demnach unter anderem die Erkenntnis zugrunde, dass sich mit Hilfe der erfindungsgemäßen cRGD-Peptid-Derivate in besonders einfacher Weise eine dünne Deckschicht auf einer Basis aus einem hydrophoben Material, die zum Beispiel einen Wirkstoff enthält, herstellen lässt. Die Deckschicht liegt in der Regel als Monolage vor, bei der der hydrophobe Rest der cRGD-Peptid-Derivate in das hydrophobe Material der Basis ragt. Hierdurch wird die Elutionscharakteristik der Basis für den Wirkstoff nicht oder allenfalls nur in sehr geringem Umfang verändert.

Das Implantat weist demnach in diesen Fällen eine aus zumindest 2 Bestandteilen bestehende Beschichtung auf, einer auf den Grundkörper aufgetragenen Basisschicht aus einem hydrophoben Material und eine diese Basisschicht zumindest partiell bedeckende Deckschicht, die aus dem cRGD-Peptid-Derivat besteht oder dieses enthält.

Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 100 µm, besonders bevorzugt 300 nm bis 15 µm. Die Beschichtung kann direkt auf die Implantatoberfläche aufgetragen werden. Die Verarbeitung kann nach Standardverfahren für die Beschichtung erfolgen. Als Basisschicht können einschichtige, aber auch mehrschichtige Systeme (zum Beispiel so genannte base coat -, drug coat - oder top coat - Schichten) erstellt werden. Die Basisschicht kann unmittelbar auf dem Grundkörper des Implantats aufgebracht sein oder es sind weitere Schichten dazwischen vorgesehen. Verfahren zur Beschichtung von Implantaten sowohl zur Erstellung der Basisschicht als auch zur Erstellung der Deckschicht sind dem Fachmann bekannt.

Unter Wirkstoff im erfindungsgemäßen Sinne wird ein Arzneistoff mit pharmazeutischer Wirkung verstanden, der im menschlichen oder tierischen Körper zur Heilung, Linderung, Verhütung oder Erkennung von Krankheiten dient. Wirkstoffe umfassen insbesondere Paclitaxel, Sirolimus, Rapamycin und Rapamycinderivate. Insbesondere Wirkstoffe, die über die mTOR Erkennungssequenz wirken. Ferner RAS Inhibitoren, insbesondere solche, die die RAS Adhäsion verhindern.

Gemäß einer weiteren bevorzugten Ausführungsform besteht die Deckschicht aus Chitosan oder Polylysin. Chitosan oder Polylysin kann in verdünnter wässriger Lösung zum Beispiel auf einen ggf. auch mit einem wirkstoffbeladenen Basismaterial vorbeschichteten Stent durch einen Sprüh- oder Tauchprozess aufgebracht werden. Die nachträgliche Ankopplung mit cRGD's geschieht dann im wässrigen Medium, in dem bei lipophilen Wirkstoffen nicht die Gefahr besteht, dass in diesem Medium die in der Basis eingebundenen Wirkstoffe eluiert und damit die Wirkstoffbeladung sinkt.

Ein weiterer Aspekt der Erfindung liegt in der Bereitstellung eines Verfahrens zur Herstellung eines obig genannten cRGD-Peptid-Derivat der Formel (1). Das Verfahren umfasst den Schritt:
kovalente Kopplung eines primären Amins der Formel R-NH₂ mit der Isothiocyanatgruppe eines cRGD-Peptid-Derivats der Formel (2)

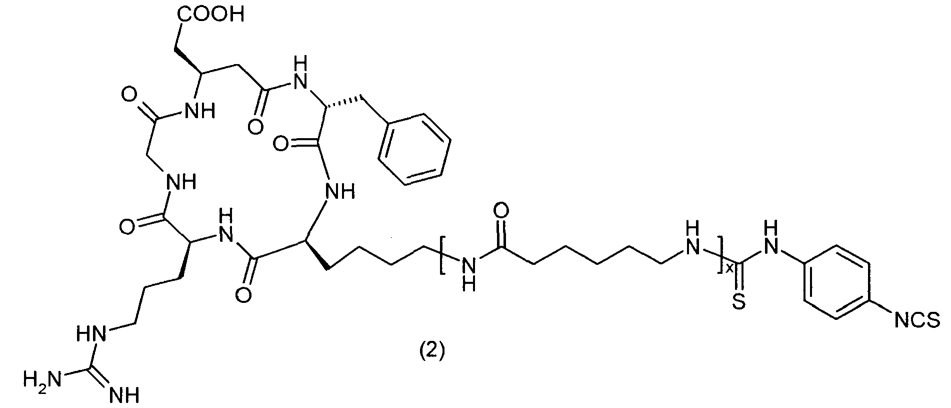

Die Verbindung der Formel (2) ist bekannt (Jörg Auernheimer; Funktionalisierung künstlicher Oberflächen mit Integrinliganden zur Stimulierung integrinvermittelter Zelladhäsion; Dissertation; TU München; 2005; Kap 6.4; 5.136). Beispiele für zur Kopplung geeignete primäre Amine umfassen 2-Amino-anthracen, 4-Heptylanilin oder 1-Aminoheptan, welche ohne Nebenreaktionen an die reaktive Gruppe ankoppeln.

### Detaillierte Beschreibung der Erfindung

Die Erfindung wird nachfolgend anhand von Ausführungsbeispielen näher erläutert.

### Umsetzung der Verbindung der Formel (2) mit 1-Aminoheptan

2 ml der Verbindung der Formel (2) mit x = 4 in einer Konzentration von 5 mg/ml in PBS Puffer werden bei Raumtemperatur mit 1,52 mg 1-Aminoheptan (logP 2,57) umgesetzt. Die so erhaltene Lösung kann auf eine wirkstofftragende Oberfläche eines Implantates, bestehend aus einem Grundkörper und einer wirkstofftragenden Matrix, aufgesprüht werden. Nach dem Trocknen sind die Aminosäureeinheiten an der Oberfläche, während die lipophile Gruppe mit der wirkstofftragenden Schicht wechselwirkt. Die Bindungsstärke ist umso größer, je lipophiler der matrixgebundene Wirkstoff ist und je lipophiler die Matrix ist.

Die Stabilität der Ankopplung kann folgendermaßen zusätzlich erhöht werden: Die Stents mit wirkstofftragender Sicht und cRGD Deckschicht werden für 30 Min. in eine Chloroformatmosphäre gebracht. Durch diesen Lösungsmitteleinfluss wird die PLLA Oberfläche angelöst, so dass die Heptangruppe in die äußere Oberfläche mit eingebaut wird. Die so immobilisierten cRGD's sind unverlierbar gebunden.

### Umsetzung der Verbindung der Formel (2) an PLLA-Oberfläche

10 ml Chloroform werden vorgelegt und mit 2 ml Wasser überschichtet. Die cRGD's (x = 4) werden in 500 µl wässriger Lösung dazugegeben (Konz.: 5mg/ml).

Der mit PLLA beschichtete Stent wird bis in die Chloroformphase getaucht und 1 - 5 Sek. bei RT inkubiert. Beim langsamen Herausziehen lagern sich die cRGD's mit ihrem lipophilen Rest an das gequollene PLLA an. Nach dem Trocknen sind die cRGD's unverlierbar gebunden. Die Peptide sind exprimiert und können mit Integrinen wechselwirken.

### Umsetzung der Verbindung der Formel (2) an Chitosan-Oberfläche

Ein Stent mit einer Beschichtung aus Polylactid (PLLA) und Sirolimus wird mit einer Lösung von Chitosan in verdünnter Essigsäure (0,3 %) besprüht. Nach dem Trocknen wird der Stent in eine gepufferte (Phosphatpuffer 50mM) wässrige Lösung eines cRGD's der Formel (2) mit x = 4 getaucht (Konzentration: 5 mg/ml; pH 7; Raumtemperatur). Nach 1h sind alle Amingruppen des Chitosan mit den Thiocyanatgruppen des cRGD's abreagiert.

Der Stent zeigt keine abweichende Elutionskinetik für Sirolimus. Im Gegensatz zur rein mit PLLA und Sirolimus beschichteten Stents ist schon 3 Tage nach Implantation eine erste Endothelbedeckung sichtbar.

## Patentansprüche

1. cRGD-Peptid-Derivat der Formel (1): 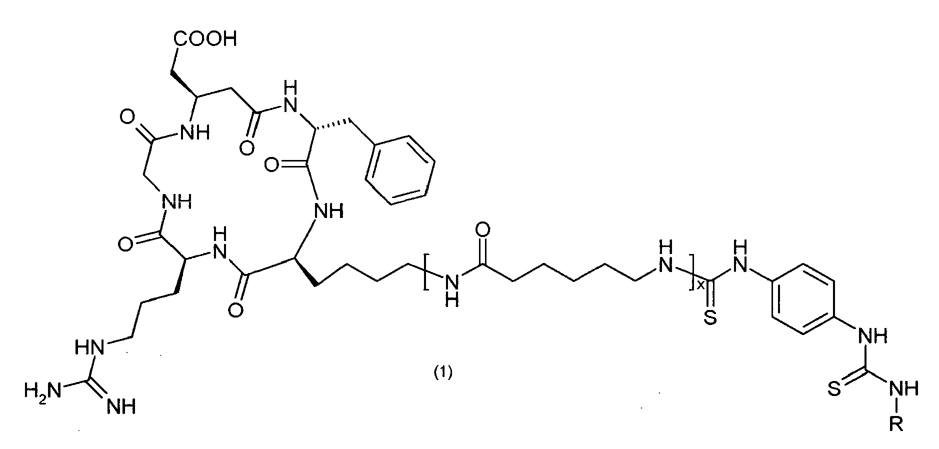 mit x = 0 - 8 und R einem hydrophoben Rest.

2. cRGD-Peptid-Derivat nach Anspruch 1, bei dem der hydrophobe Rest R derart beschaffen ist, dass ein den gleichen Rest aufweisendes primäres Amin R-NH₂ einen logP-Wert von größer als -0.2 aufweist.

3. cRGD-Peptid-Derivat nach Anspruch 1, bei dem der logP-Wert des primären Amins R-NH₂ größer als 1.9 ist.

4. cRGD-Peptid-Derivat nach Anspruch 1, bei dem x = 4 - 8 ist.

5. cRGD-Peptid-Derivat nach Anspruch 1, bei dem R für einen Rest steht ausgewählt aus der Gruppe umfassend substituierte oder unsubstituierte aliphatische Kohlenwasserstoffe; substituierte oder unsubstituierte heteroaliphatische Kohlenwasserstoffe mit wenigstens einen Heteroatom ausgewählt aus N, O, S oder P; substituierte oder unsubstituierte Aromaten; und substituierte oder unsubstituierte N-, O-, S- oder P-Heteroaromaten.

6. cRGD-Peptid-Derivat nach Anspruch 1, bei dem R für eine polymere Gruppe, ausgewählt aus Chitosan, Polylysin, Polylactid-co-glycolid (PLGA) und Polylactid (PLA) steht.

7. Implantat mit einer Beschichtung, die ein cRGD-Peptid-Derivat nach einem der Ansprüche 1 bis 6 enthält.

8. Implantat nach Anspruch 7, bei dem die Beschichtung umfasst:
(i) eine Basis aus einem hydrophoben Material; und
(ii) eine Deckschicht, die ein cRGD-Peptid-Derivat enthält.

9. Implantat nach Anspruch 8, bei dem das hydrophobe Material der Basis Polylactidco-glycolid (PLGA) oder Polylactid (PLA) enthält und das cRGD-Peptid-Derivat in der Deckschicht einen hydrophoben polymeren Rest aus Polylactid-co-glycolid (PLGA) beziehungsweise Polylactid (PLA) aufweist.

10. Implantat nach Anspruch 8, bei dem die Deckschicht einen hydrophoben polymeren Rest aus Chitosan oder Polylysin aufweist.

11. Implantat nach einem der Ansprüche 8 bis 10, bei dem das hydrophobe Material einen Wirkstoff enthält.

12. Implantat nach einem der Ansprüche 7 bis 11, bei dem das Implantat ein Stent ist.

13. Verfahren zur Verstellung eines cRGD-Peptid-Derivats der Formel (1): 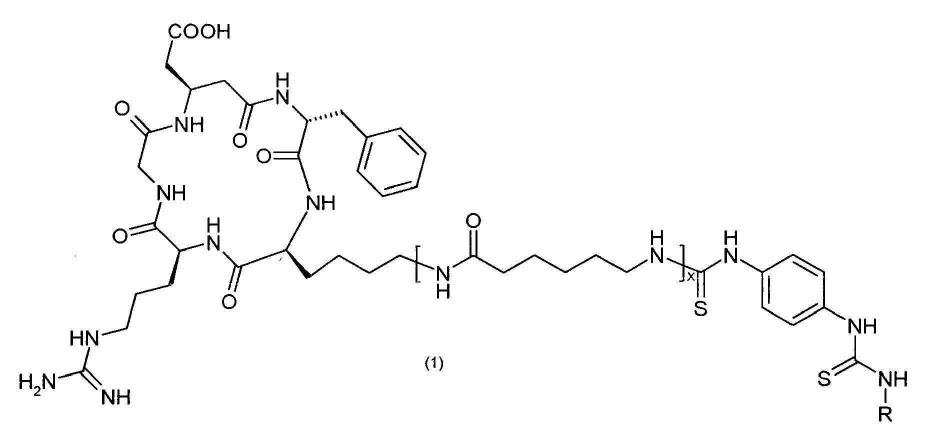 mit x = 0 - 8 ist und R für einen hydrophoben Rest steht, wobei das Verfahren den Schritt umfasst:
kovalente Kopplung eines primären Amins der Formel R-NH₂ mit der Isothiocyanatgruppe eines cRGD-Peptid-Derivats der Formel (2)

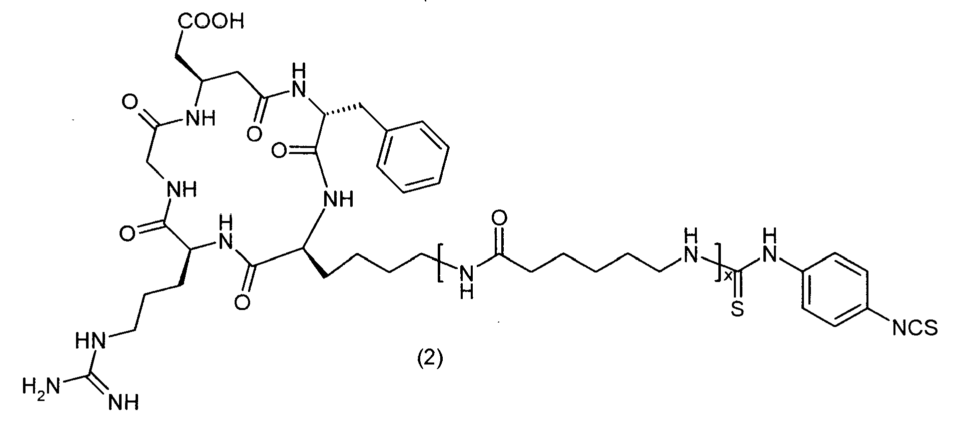

## Claims

1. A cRGD peptide derivative having the formula (1): 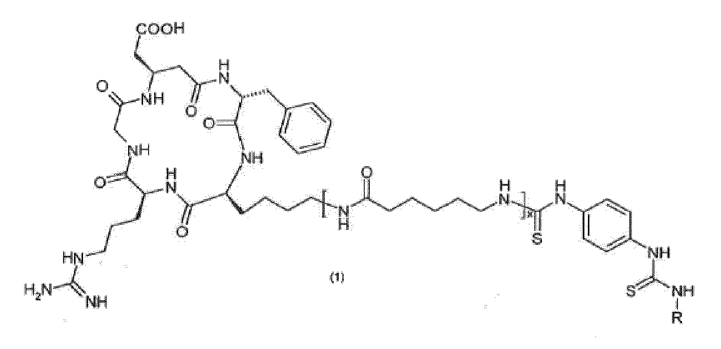 where x = 0 to 8 and R is a hydrophobic group.

2. The cRGD peptide derivative according to claim 1, wherein the hydrophobic group R is constituted such that a primary amine R-NH₂ comprising the same group has a logP value of greater than -0.2.

3. The cRGD peptide derivative according to claim 1, wherein the logP value of the primary amine R-NH₂ is greater than 1.9.

4. The cRGD peptide derivative according to claim 1, wherein x = 4 to 8.

5. The cRGD peptide derivative according to claim 1, wherein R is a group selected from the group consisting of substituted or unsubstituted aliphatic hydrocarbons, substituted or unsubstituted heteroaliphatic hydrocarbons having at least one heteroatom selected from N, O, S or P, substituted or unsubstituted aromatic compounds, and substituted or unsubstituted N, O, S or P heteroaromatic compounds.

6. The cRGD peptide derivative according to claim 1, wherein R is a polymeric group selected from chitosan, polylysine, polylactide-co-glycolide (PLGA) and polylactide (PLA).

7. An implant having a coating comprising a cRGD peptide derivative according to any one of claims 1 to 6.

8. The implant according to claim 7, wherein the coating comprises:
(i) a base made of hydrophobic material; and
(ii) a top coat comprising a cRGD peptide derivative.

9. The implant according to claim 8, wherein the hydrophobic material of the base comprises polylactide-co-glycolide (PLGA) or polylactide (PLA), and the cRGD peptide derivative in the top coat comprises a hydrophobic polymeric residue made of polylactide-co-glycolide (PLGA) or polylactide (PLA).

10. The implant according to claim 8, wherein the top coat comprises a hydrophobic polymeric residue made of chitosan or polylysine.

11. An implant according to any one of claims 8 to 10, wherein the hydrophobic material comprises an active ingredient.

12. An implant according to any one of claims 7 to 11, wherein the implant is a stent.

13. A method for producing a cRGD peptide derivative having the formula (1): 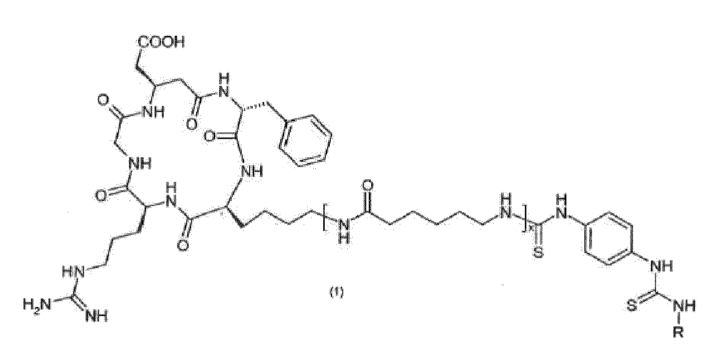 where x = 0 to 8 and R is a hydrophobic group, wherein the method comprises the following step:
covalently coupling a primary amine having the formula R-NH₂ with the isothiocyanate group of a cRGD peptide derivative having the formula (2)

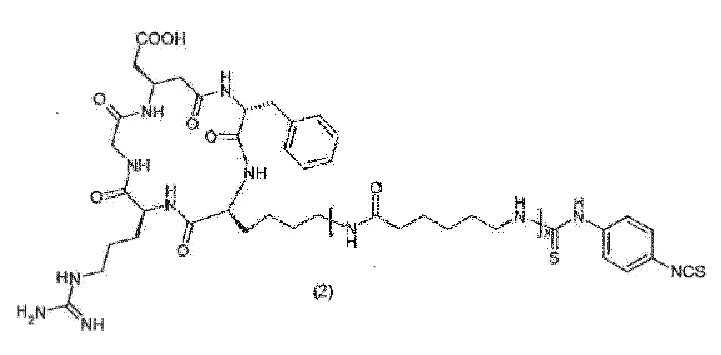

## Revendications

1. Dérivé de peptide cRGD de formule (1) : 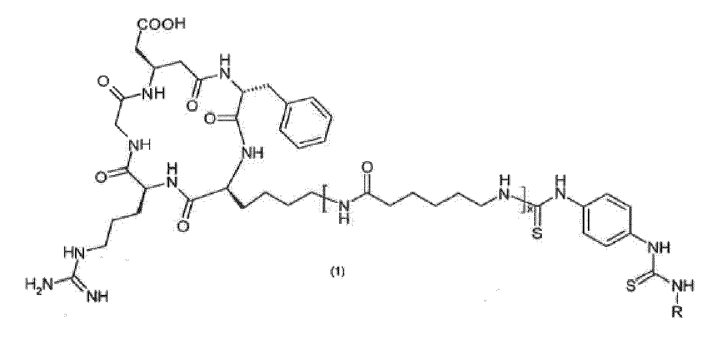 où x = 0 à 8 et R représente un groupement hydrophobe.

2. Dérivé de peptide cRGD conforme à la revendication 1, où le groupement hydrophobe R est constitué de telle sorte qu'une amine primaire R-NH₂ comportant ledit groupement présente une valeur de logP supérieure à -0,2.

3. Dérivé de peptide cRGD conforme à la revendication 1, où la valeur de logP de l'amine primaire R-NH₂ est supérieure à 1,9.

4. Dérivé de peptide cRGD conforme à la revendication 1, où x = 4 à 8.

5. Dérivé de peptide cRGD conforme à la revendication 1, où R représente un groupement choisi dans le groupe constitué par les hydrocarbures aliphatiques substitués ou non substitués, les hydrocarbures hétéroaliphatiques substitués ou non substitués comportant au moins un hétéroatome choisi parmi N, O, S et P, les composés aromatiques substitués ou non substitués et les composés N-, O-, S- ou P-hétéroaromatiques substitués ou non substitués.

6. Dérivé de peptide cRGD conforme à la revendication 1, où R représente un groupement polymère choisi parmi le chitosane, la polylysine, le polylactide-co-glycolide (PLGA) et le polylactide (PLA).

7. Implant portant un revêtement comprenant un dérivé de peptide cRGD conforme à l'une quelconque des revendications 1 à 6.

8. Implant conforme à la revendication 7, où le revêtement comprend :
(i) un support fabriqué en un matériau hydrophobe ; et
(ii) une couche supérieure comprenant un dérivé de peptide cRGD.

9. Implant conforme à la revendication 8, où le matériau hydrophobe du support comprend du polylactide-co-glycolide (PLGA) ou du polylactide (PLA), et le dérivé de peptide cRGD de la couche supérieure comprend un résidu polymère hydrophobe de polylactide-co-glycolide (PLGA) ou de polylactide (PLA).

10. Implant conforme à la revendication 8, où la couche supérieure comprend un résidu polymère hydrophobe fabriqué en chitosane ou en polylysine.

11. Implant conforme à l'une quelconque des revendications 8 à 10, où le matériau hydrophobe comprend un principe actif.

12. Implant conforme à l'une quelconque des revendications 7 à 11, où l'implant est un stent.

13. Procédé de production d'un dérivé de peptide cRGD de formule (1) : 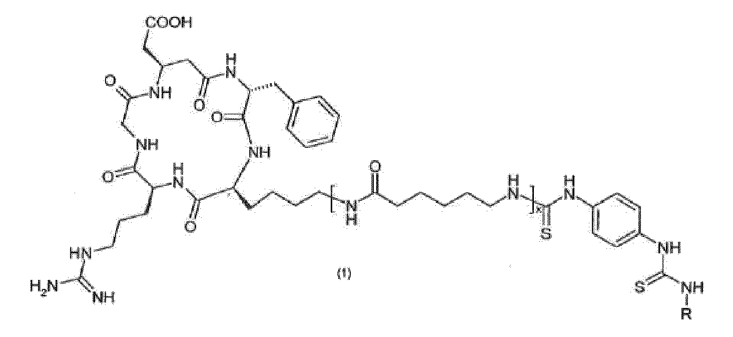 où x = 0 à 8 et R représente un groupement hydrophobe, où le procédé comprend l'étape suivante :
couplage covalent d'une amine primaire de formule R-NH₂ avec le groupement isothiocyanate d'un dérivé de peptide cRGD répondant à la formule (2)
